(19) Europäisches Patentamt
European Patent Office
Office européen des brevets

(11) EP 1 754 473 A1

(12) EUROPEAN PATENT APPLICATION
published in accordance with Art. 158(3) EPC

(43) Date of publication:
21.02.2007 Bulletin 2007/08

(21) Application number: 05743726.1

(22) Date of filing: 30.05.2005

(51) Int Cl.:
*A61K 31/047* (2006.01) *A61P 35/00* (2006.01)

(86) International application number:
PCT/JP2005/009895

(87) International publication number:
WO 2005/115364 (08.12.2005 Gazette 2005/49)

(84) Designated Contracting States:
AT BE BG CH CY CZ DE DK EE ES FI FR GB GR HU IE IS IT LI LT LU MC NL PL PT RO SE SI SK TR

(30) Priority: 31.05.2004 JP 2004162641
24.08.2004 JP 2004243836
05.10.2004 JP 2004292536

(71) Applicant: Takara Bio, Inc.
Otsu-shi,
Shiga 520-2193 (JP)

(72) Inventors:
- KAWAI, Takashi
TAKARA BIO INC.
Shiga 520-2193 (JP)
- MIZUTANI, Shigetoshi
TAKARA BIO INC.
Shiga 520-2193 (JP)
- ENOKI, Tatsuji
TAKARA BIO INC.
Shiga 520-2193 (JP)
- SAGAWA, Hiroaki
TAKARA BIO INC.
Shiga 520-2193 (JP)
- SAKAI, Takeshi
TAKARA BIO INC.
Shiga 520-2193 (JP)
- SHIMANAKA, Kazuo
TAKARA BIO INC.
Shiga 520-2193 (JP)
- KATO, Ikunoshin
TAKARA BIO INC.
Shiga 520-2193 (JP)

(74) Representative: Vossius & Partner
Siebertstrasse 4
81675 München (DE)

# (54) ANTITUMOR AGENT

(57) According to the present invention, an antitumor agent, an apoptosis-inducing agent or an angiogenesis-suppressing agent having the effect even via an oral route, containing a compound contained in an inexpensive edible basidiomycete or a composition derived from an edible basidiomycete, and a food having the antitumor activity, the apoptosis-inducing activity or the angiogenesis-suppressing activity containing the compound or the composition can be obtained. Therefore, the present invention is particularly useful, especially in the field of a medicine and a health food.

[Fig. 1]

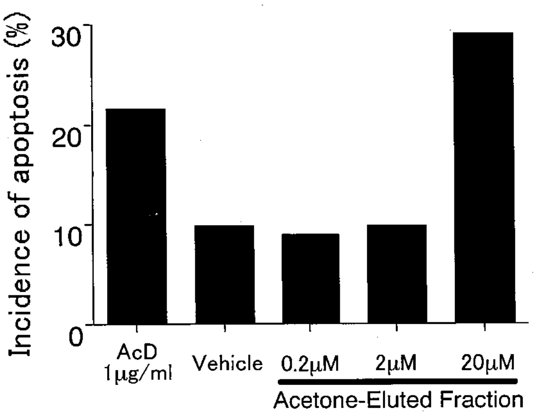


EP 1 754 473 A1

## Description

### TECHNICAL FIELD

The present invention relates to an antitumor agent, an apoptosis-inducing agent or an angiogenesis-suppressing agent containing a mushroom-derived physiologically active ingredient as an active ingredient, and a food containing the same.

### BACKGROUND ART

It is widely known that various edible basidiomycetes such as a shiitake mushroom *(Lentinus edodes),* a hen of the woods (*Grifola frondosa*), or a velvet shank *(Flammulina velutipes)* have antitumor properties, and various antitumor compositions have been prepared from a fruit body or a mycelium thereof. In addition, it has been revealed that a bunashimeji mushroom (*Lyophyllum ulmarium*), which is one of edible basidiomycetes, has antitumor properties, and for example, it has been revealed that a hot-water extract of a fruit body having a protein content of 3 to 20% and a sugar content of 20 to 50%, and a purified polysaccharide therefrom (e.g. Patent Publication 1), and further, a physiologically active substance EEM-S having a molecular weight of 6,000 to 60,000, which is obtained by suspending the mushroom in water or a hydrophilic solvent, and subjecting the resulting suspension to heat treatment, have the antitumor activity (e.g. Patent Publication 2).

It is known that, when a malignant tumor is formed and grows, angiogenesis is induced. Induction of angiogenesis leads to transportation of nutrients to the tumor, which causes rapid growth of tumor cells and malignancy, or to invasion into a vessel and migration to various tissues of tumor cells, which easily causes metastasis. That is, it is thought that, when angiogenesis is suppressed, proliferation, conversion into a malignant state and metastasis of tumor cells can be suppressed. Based on such conception, development of a medicine has been progressed, but nothing has been merchandized yet.

On the other hand, there are numerous reports regarding a substance inducing apoptosis of tumor cells. However, an apoptosis-inducing substance may also target endothelial cells of a newly formed blood vessel having a high proliferating ability. There is also a report on a substance suppressing a tumor which has been developed from such a viewpoint (e.g. Non-Patent Publication 2). Since such a substance has a weak action of causing apoptosis on tumor cells themselves but has an effect of suppressing angiogenesis induced by tumor cells, leading to so-called starvation policy, it is presumed that the substance has few side effects.

It has been reported that some components derived from a mushroom also have an angiogenesis-suppressing action. An example of the component is ergosterol derived from a himematsutake mushroom *(Agaricus blazei* Murill) (Non-Patent Publication 3).

A terpene compound (terpenoid), which is an organic compound present in many plants, has carbon atoms in a multiple of 5, and is derived from a precursor composed of isoprenes or isopentanes of the number of n. A terpene compound of n=2 is termed monoterpene, that of n=3 is termed sesquiterpene, that of n=4 is termed diterpene, that of n=5 is termed sesterterpene, that of n=6 is termed triterpene, that of n=8 is termed tetraterpene, and that of n= 9 or more is termed polyterpene. A terpene compound is known to have an action of inducing apoptosis, and there are many reports of the apoptosis-inducing action of, for examples, triterpene.

As a physiologically active component derived from *Lyophyllum ulmarium,* it is known that a substance termed SBS has an action of suppressing platelet aggregation and an action of an anti-carcinogenesis promoter (Patent Publication 3). In addition, it is known that *Lyophyllum ulmarium* contains various polyterpenes which are bitter components (e.g. Non-Patent Publication 1).

Patent Publication 1: JP-A-Hei-5-306233
Patent Publication 2: WO 01/51070
Patent Publication 3: JP-A-Hei-4-104795
Non-Patent Publication 1: Sawabe A. and three others, Journal of Mass Spectrometry, 31: 921-925 (1996)
Non-Patent Publication 2: Schwarz R.E. and one other, J. Surg. Res., 120(1): 64-72 (2004)
Non-Patent Publication 3: Takaku T. and two others, J. Nutr., 131(5): 1409-1413 (2001)

### DISCLOSURE OF THE INVENTION

### PROBLEMS TO BE SOLVED BY THE INVENTION

It has been revealed that a fruit body of various edible basidiomycetes has an antitumor property, but in order

to obtain the effect, it is necessary to eat routinely a constant amount of the fruit body. However, since even an edible basidiomycete has a peculiar taste, it is difficult to eat routinely a constant amount of the fruit body even when the way to cook the fruit body is devised.
On the other hand, although many health foods with which an active ingredient having an antitumor property, an immunopotentiating activity or the like derived from a certain basidiomycete is blended are widely distributed in markets, a health food produced therefrom inevitably is very expensive, since basidiomycete as a raw material is rare.
An object of the present invention is to provide a medicine or a food exhibiting the effect even via an oral route, utilizing a compound having an antitumor activity derived from an inexpensive edible basidiomycete.

MEANS TO SOLVE THE PROBLEMS

As a result of extensive studies to solve the aforementioned problems, the present inventors found that polyterpene derived from a fruit body of *Lyophyllum ulmarium* has an antitumor activity, an apoptosis-inducing activity and an angiogenesis-suppressing activity, which resulted in completion of the present invention.
That is, to summarize the present invention, a first invention of the present invention relates to an antitumor agent, an apoptosis-inducing agent or an angiogenesis-suppressing agent, characterized in that the agent contains as an active ingredient at least one substance selected from the group consisting of a compound represented by the following general formula (1), a derivative thereof, and a pharmacologically acceptable salt thereof,
**[0012]**

[Chemical Formula 1]

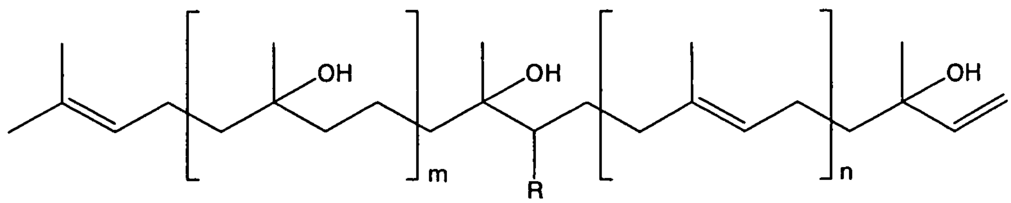

(1)

wherein **m** represents an integer of **1** to 9, **n** represents an integer of 1 to 7, provided that **m** + **n=5** to 11; and **R** represents a hydrogen atom or a hydroxyl group.
A second invention of the present invention relates to the antitumor agent, the apoptosis-inducing agent or the angiogenesis-suppressing agent of the first invention, wherein the compound represented by the general formula **(1)** is at least one compound represented by the following formulas (2) to (11).
[Chemical Formula 2]

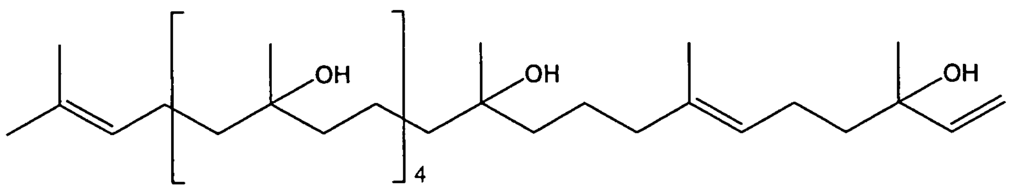

(2)

[Chemical Formula 3]

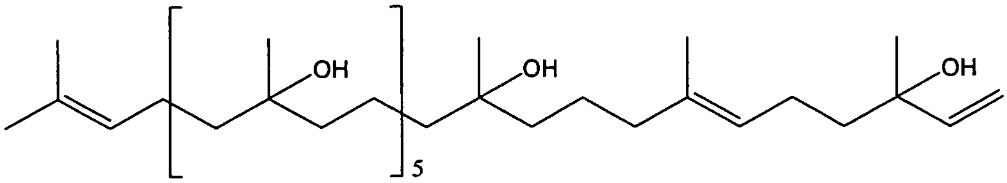

(3)

[Chemical Formula 4]

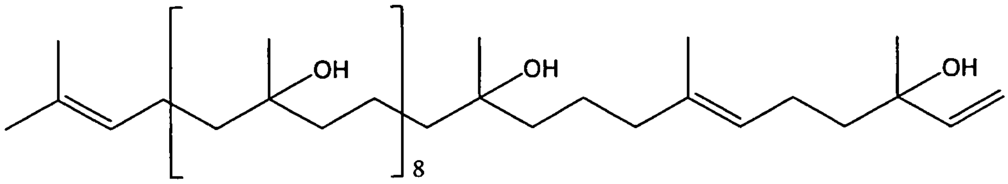

(4)

[Chemical Formula 5]

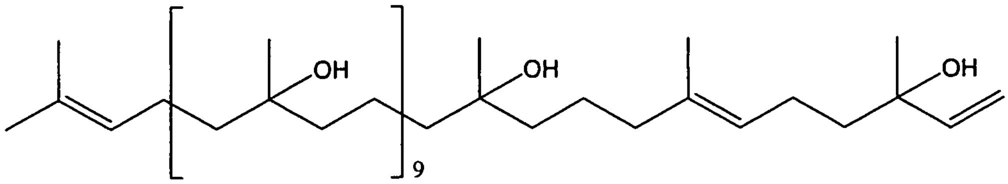

(5)

[Chemical Formula 6]

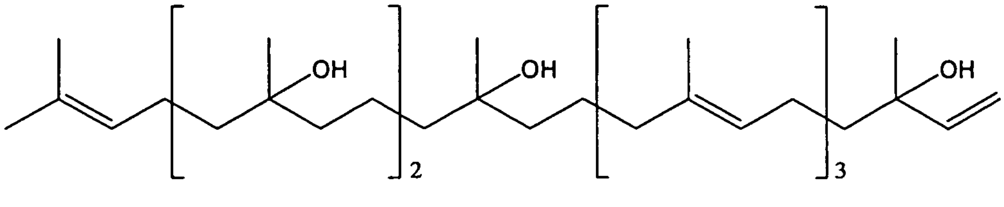

(6)

[Chemical Formula 7]

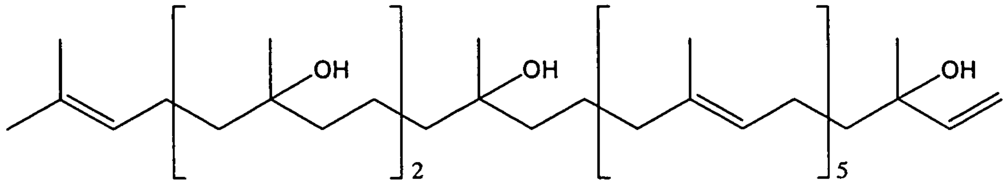

(7)

[Chemical Formula 8]

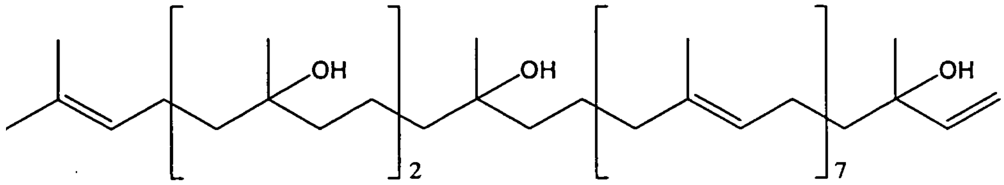

(8)

[Chemical Formula 9]

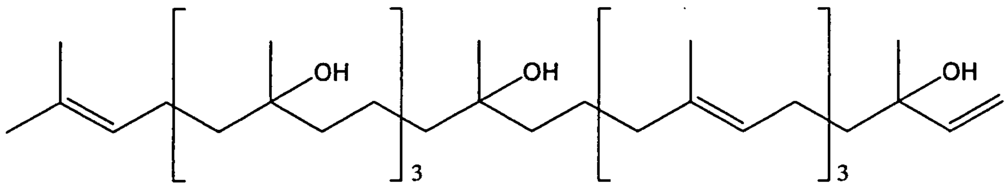

(9)

[Chemical Formula 10]

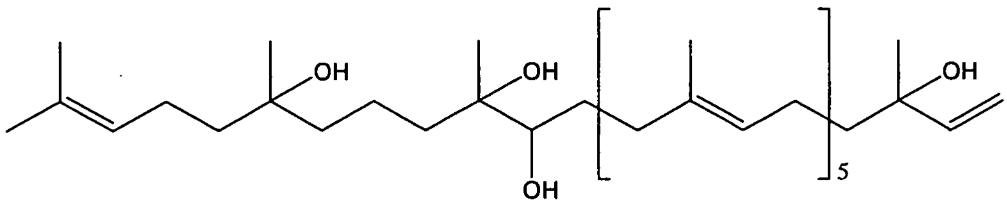

(10)

[Chemical Formula 11]

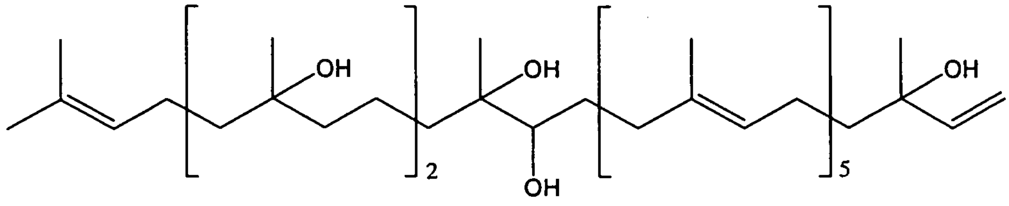

(11)

A third invention of the present invention relates to a food having an antitumor activity, an apoptosis-inducing activity or an angiogenesis-suppressing activity, characterized in that the food contains at least one substance selected from the group consisting of a compound represented by the general formula (1), a derivative thereof, and a pharmacologically acceptable salt thereof.

A fourth invention of the present invention relates to the food of the third invention, wherein the compound represented by the general formula (1) is at least one compound represented by the formulas (2) to (11).

A fifth invention of the present invention relates to an antitumor agent, an apoptosis-inducing agent or an angiogenesis-suppressing agent, characterized in that the agent contains as an active ingredient a *Lyophyllum ulmarium-derived* composition containing at least one substance selected from the group consisting of compounds represented by the formulas (2) to (11), a derivative thereof, and a pharmacologically acceptable salt thereof.

A sixth invention of the present invention relates to the antitumor agent, the apoptosis-inducing agent or the angiogenesis-suppressing agent of the fifth invention, characterized in that the *Lyophyllum ulmarium*-derived composition is a composition obtained by adsorbing an ethyl-acetate extract of *Lyophyllum ulmarium* onto a silica gel column, followed by elusion.

A seventh invention of the present invention relates to a food having an antitumor-activity, an apoptosis-inducing activity or an angiogenesis-suppressing activity, characterized in that the food contains a *Lyophyllum ulmarium*-derived composition containing at least one substance selected from the group consisting of compounds represented by the formulas (2) to (11), a derivative thereof, and a pharmacologically acceptable salt thereof.

An eighth invention of the present invention relates to the food of the seventh invention, characterized in that the *Lyophyllum ulmarium*-derived composition is a composition obtained by adsorbing an ethyl-acetate extract of *Lyophyllum ulmarium* onto a silica gel column, followed by elusion.

A ninth invention of the present invention relates to a *Lyophyllum ulmarium-derived* composition containing at least one substance selected from the group consisting of compounds represented by the formulas (2) to (11), a derivative thereof and a pharmacologically acceptable salt thereof, wherein the composition is obtained by adsorbing an ethyl-acetate extract of *Lyophyllum ulmarium* onto a silica gel column, followed by elusion.

A tenth invention of the present invention relates to a method of treating a disease in a subject in need of an antitumor action, an apoptosis-inducing action or an angiogenesis-suppressing action, comprising administering to the subject an effective amount of at least one substance selected from the group consisting of a compound represented by the aforementioned general formula (1), a derivative thereof, and a pharmacologically acceptable salt thereof.

An eleventh invention of the present invention relates to use of at least one substance selected from the group consisting of a compound represented by the aforementioned general formula (1), a derivative thereof, and a pharmacologically acceptable salt thereof, for manufacturing an antitumor agent, an apoptosis-inducing agent or an angiogenesis-suppressing agent.

## EFFECTS OF THE INVENTION

According to the present invention, an antitumor agent, an apoptosis-inducing agent or an angiogenesis-suppressing agent having the effect even via an oral route, containing a compound contained in an inexpensive edible basidiomycete or a composition derived from an edible basidiomycete, and a food having the antitumor activity, the apoptosis-inducing activity or the angiogenesis-suppressing activity containing the compound or the composition can be obtained.

## BRIEF DESCRIPTION OF THE DRAWINGS

[Fig.1] Fig. 1 is a graph showing the apoptosis-inducing action of an acetone-eluted fraction when HL-60 cells

are cultured at 37°C for 6 hours in an RPMI-1640 medium with no FCS added thereto. In the figure, Vehicle represents a 1% aqueous ethanol solution.

BEST MODE FOR CARRYING OUT THE INVENTION

*Lyophyllum ulmarium* grows in clusters or separately grows on a withered tree of various broad-leaved trees in autumn in nature, and since *Lyophyllum ulmarium* has a better shape and a crunchy texture as compared with other mushrooms, it has been eaten as a mushroom of a good taste. In addition, in recent years, an artificial cultivating method using a bacterial bed for cultivation in a bottle or a box using a culture medium wherein bran or other nutrient source is blended with sawdust has been established, and it has become possible to stably harvest a mushroom throughout a year regardless of the season. That is, *Lyophyllum ulmarium* as a raw material of an active ingredient in the present invention can be obtained at a low cost, and is also suitable as a raw material of a medicine or a health food.

*Lyophyllum ulmarium* used as a raw material may be a natural or artificially cultivated product, and preferably, it can be exemplified by *Lyophyllum ulmarium* M-8171 (FERM BP-1415, deposit date: August 23, 1986) or *Lyophyllum ulmarium* K-0259 (FERM P-12981, deposit date: June 2, 1992), (these fungus were both deposited at International Patent Organism Depositary, Independent Administrative Agency National Institute of Advanced Industrial Science and Technology (AIST Tsukuba Central 6, 1-1, Higashi 1-chome, Tsukuba-shi, Ibaraki-ken, Japan, zip code: 305-8566)). In addition, these fungus are distributed in markets under the trade name "YAMABIKO HONSHIMEJI (registered trade mark)" or "SUPER YAMABIKO SHIMEJI (registered trade mark)." A fruit body may be raw, or a dried fruit body obtained by drying such as drying by heating, sun drying, lyophilization and the like. Alternatively, regarding a fruit body, a bunch as it is or a ground fruit body may be used as a raw material. Alternatively, as a raw material, a mycelium of *Lyophyllum ulmarium* or a lyophilized product thereof may also be used.

The present inventors have searched for a substance having the antitumor activity derived from *Lyophyllum ulmarium,* and as a result, found that polyterpene represented by the general formula (1) has a strong antitumor activity, apoptosis-inducing activity and angiogenesis-suppressing activity, the effect of which is exhibited even via an oral route.

The compound represented by the general formula (1) used in the present invention is exemplified by compounds represented by the formulas (2) to (11), and particularly preferably, from a viewpoint of a large content in a fruit body of *Lyophyllum ulmarium* and ease in production, a compound represented by the formula (3) is used.

The compound represented by the formula (3) which is a part of an active ingredient of the present invention is the same compound as a novel substance SBS described in JP-A-Hei-4-104795 (hereinafter, the compound represented by the formula (3) is referred to as SBS in some cases), and the patent describes that, since SBS has the action of suppressing Epstein-Barr virus activation, the compound has an anti-carcinogenesis promoter action. However, the gazette does not disclose that an antitumor activity, an apoptosis-inducing activity and an angiogenesis-suppression activity as in the present invention are exhibited by oral administration. That is, while JP-A-Hei-4-104795 was made on the basis of the findings of a prophylactic activity on development of a tumor, the present invention was made on the basis of the findings that SBS has effects of treating cancer and suppressing progression or metastasis of cancer by oral administration as shown in Example 1 described later, and relates to novel use of SBS. In addition, regarding a chemical structure of SBS, the present inventors analyzed the structure in Examples described later, and it was found that SBS was the same substance as hypsiziprenol $A_9$ described in the publication of Sawabe et al. (Sawabe A. and three others, Journal of Mass Spectrometry, 31: 921-925 (1996)).

A process for producing a compound represented by the formula (1) used in the present invention is not particularly limited, and the compound can be produced by a known process such as chemical synthesis and extraction from a natural product (e.g. the aforementioned *Lyophyllum ulmarium),* and for example, when SBS is produced, the process described in JP-A-Hei-4-104795 can be applied, and when compounds represented by the formulas (2) to (11) are produced, the compounds may also be produced according to the process of the aforementioned publication of Sawabe et al. Alternatively, compounds represented by the formulas (2) to (11) may be produced by various chromatographies based on the resulting extract according to the process described in following Preparation Example 1.

A derivative of the compound represented by the general formula (1) used in the present invention includes, for example, a derivative (prodrug) which can be easily hydrolyzed in a living body to exhibit desired effects, such as an ester. In addition, a derivative obtained by metabolizing the compound of the present invention administered to a mammal is also included in the derivative of the present invention. Such a prodrug may be prepared according to a known process. Such a derivative may be a salt thereof.

In addition, as a salt of the compound represented by the general formula (1) or a derivative thereof, a pharmacologically acceptable salt is used. Alternatively, a derivative of the compound which can function as a prodrug may be used. Alternatively, various isomers such as an optical isomer, a keto-enol tautomer, or a geometrical isomer of the compound used in the present invention, and an isolate of each isomer can be all used in the present invention as far as they have an antitumor activity, an apoptosis-inducing activity or an angiogenesis-suppressing activity.

The pharmacologically acceptable salt of the compound represented by the general formula (1) is exemplified

by an alkali metal salt, an alkaline earth metal salt, and a salt with an organic base. As used herein, the pharmacologically acceptable salt means a salt of a compound which is substantially atoxic to an organism, and which has an antitumor activity, an apoptosis-inducing activity, or an angiogenesis-suppressing activity. The salt includes, for example, salts of sodium, potassium, calcium, magnesium, ammonium and protonated benzathine (N, N'-di-benzylethylenediamine), choline, ethanolamine, diethanolamine, ethylenediamine, meglamine (N-methylglucamine), benethamine (N-benzylphenethylamine), piperazine, tromethamine (2-amino-2-hydroxymethyl-1,3-propanediol) and the like. Such salts may also be prepared according to known methods.

In addition, in the fifth to eighth inventions of the present invention, a *Lyophyllum ulmarium*-derived composition containing at least one substance selected from the group consisting of the compounds represented by the aforementioned formulas (Chemical Formula 2) to (Chemical Formula 11), a derivative thereof and a pharmacologically acceptable salt thereof is used. The composition is not particularly limited, and exemplified by a composition which can be obtained by adsorbing an ethyl-acetate extract of *Lyophyllum ulmarium* onto a silica gel column, and eluting the extract with a suitable eluting solvent.

As an extracting procedure for obtaining the aforementioned ethyl-acetate extract of *Lyophyllum ulmarium,* for example, when a fresh fruit body of *Lyophyllum ulmarium* is used, ethyl acetate can be used in an amount of preferably 1 to 10 parts by weight, and more preferably 1 to 5 parts by weight relative to 1 part by weight of a fresh fruit body. When a dried fruit body is used, ethyl acetate can be used in an amount of preferably 5 to 50 parts by weight, and more preferably 10 to 25 parts by weight relative to 1 part by weight of a dried product. Ethyl acetate has a low boiling point, and moreover, is used as an extracting solvent for a food, and is excellent also in safety. An extracting temperature is not particularly limited, and exemplified by 5°C to a temperature near boiling temperature of ethyl acetate (77.1°C), preferably 10° to 70°C, and further preferably 15° to 65°C. An extracting time is not particularly limited, and exemplified by 30 minutes to 24 hours, preferably 1 to 20 hours, and further preferably 2 to 10 hours. Extracting treatment may be performed under allowing to stand, or under stirring.

Removal of insolubles from the thus obtained ethyl-acetate extract may be performed by a conventional method, and for example, the insolubles can be removed by filtration or centrifugation. Here, insolubles, in the case of filtration, refers to insoluble components in an extract obtained by filtration with a commercially available filter paper (e.g. No. 2 filter manufactured by Advantec) and, in the case of centrifugation, refers to insoluble components in an extract which can be precipitated with centrifugal force of at least 5000 $\times$ g.

A silica gel column is not particularly limited, and, for example, a column charged with silica gel having a pore size of 0.063 to 0.200 mm can be used. The aforementioned ethyl-acetate extract is applied thereto. Thereafter, if necessary, the column is washed with ethyl acetate, and the extract can be eluted with various solvents. An eluting solvent is not particularly limited, and, for example, acetone can be used.

The resulting eluate can be used as it is as a composition of the present invention, and, for example, the composition of the present invention can be obtained by drying the eluate to solidness under reduced pressure, redissolving the dried product in ethanol, and further performing drying to solidness under reduced pressure. The content of the compounds represented by the formulas (2) to (11) in the composition is not particularly limited, and is preferably 0.001 to 100% by weight, and more preferably 0.01 to 10% by weight. In order to adjust the compounds represented by the formulas (2) to (11) to such a concentration, an excipient such as dextrin may be added, without limitation. Inter alia, the content of the compound represented by the formula (3) in the composition is preferably 0.0001 to 10% by weight, and more preferably 0.001 to 1 % by weight.

In addition, the compound represented by the general formula (1), a derivative thereof, and a pharmacologically acceptable salt thereof or the aforementioned *Lyophyllum ulmarium*-derived composition used in the antitumor agent, the apoptosis-inducing agent or the angiogenesis-suppressing agent of the present invention are referred to as an active ingredient of the present invention, and an antitumor agent, an apoptosis-inducing agent or an angiogenesis-suppressing agent containing the active ingredient of the present invention is referred to as a medicine of the present invention, in some cases.

No toxicity is observed in an active ingredient of the present invention, as described later. In addition, there is no fear of generation of the side effect. For this reason, the antitumor activity, the apoptosis-inducing activity and the angiogenesis-suppressing activity can be exhibited safely and suitably.

In addition, in the present invention, a disease to be treated with an antitumor agent is not particularly limited, as far as it is a disease requiring an antitumor action in treatment. The aforementioned disease is exemplified by a general malignant tumor such as stomach cancer, large intestine cancer, esophagus cancer, skin cancer, uterine cancer, prostate cancer, bladder cancer or lung cancer. Further, a malignant tumor of a hemopoietic organ such as leukemia can also be a disease to be treated with the antitumor agent of the present invention. The antitumor activity can be measured, for example, by adding an agent to a cultured cancer cell, culturing the cell for a predetermined time, and determining a survival ratio of a cancer cell, or by transplanting a cancer cell to an experimental animal such as a mouse, administering an agent for a predetermined period, and measuring size and weight of a solid tumor generated in the body of the experimental animal, and the like.

In addition, in the present invention, a disease to be treated with an apoptosis-inducing agent is not particularly limited, as far as it is a disease requiring an apoptosis-inducing action in treatment. Examples of the disease include, in addition to the aforementioned general malignant tumor, an autoimmune disease such as Sjögren syndrome. Further, a malignant tumor of a hemopoietic organ such as leukemia can also be a disease to be treated with the apoptosis-inducing agent of the present invention. Since the apoptosis-inducing activity in the present invention cannot be confirmed when a normal mouse cell is used, the apoptosis-inducing agent of the present invention having such an activity has extremely high utility on the aforementioned diseases. The apoptosis-inducing activity can be measured by adding an agent to a culturing cell, culturing the cell for a predetermined period, staining a nucleus with propidium iodide or the like, and observing the cell under a microscope or using a flow cytometer or the like.

In addition, in the present invention, a disease to be treated with the angiogenesis-suppressing agent is not particularly limited, as far as it is a disease requiring an angiogenesis-suppressing action in treatment. Examples of the disease include, in addition to the aforementioned general malignant tumor, rheumatoid arthritis, psoriasis, osteoarthritis, age-related macular degeneration, angioma, hypertrophic scar, periodontal disease, scleroderma and the like. In addition, the angiogenesis-suppressing activity can be assessed by a method of measuring the amount of hemoglobin of erythrocytes infiltrating into a Matrigel (extracellular matrix) using an absorptiometer in a model in which a Matrigel with a fibroblast growth factor or the like which induces angiogenesis added thereto is subcutaneously transplanted to a mouse.

The medicine of the present invention includes a medicine obtained by formulating the aforementioned active ingredient of the present invention into a preparation in combination with a known pharmaceutical carrier. In the medicine of the present invention, as a salt as an active ingredient, a pharmacologically acceptable salt is used. In addition, as the medicine of the present invention, the aforementioned active ingredient may be blended with or used in combination with other ingredient which can be used in the same utility as that of the active ingredient, for example, a known antitumor agent, apoptosis-inducing agent or angiogenesis-suppressing agent.

The medicine of the present invention is normally produced by blending the aforementioned active ingredient with a pharmaceutically acceptable liquid-or solid-carrier, and optionally, a solvent, a dispersant, an emulsifier, a buffer, a stabilizer, an excipient, a binder, a disintegrant, a lubricant or the like may be added thereto to obtain a solid preparation such as a tablet, granule, powder, epipastic, or a capsule, or a liquid preparation such as a common solution, a suspension, or an emulsion. Alternatively, the medicine may be in the form of a dry product which can be formed into liquid by addition of a suitable carrier prior to use, or external use preparations.

A pharmaceutical carrier can be selected depending on an administration form and a dosage form of the medicine of the present invention. When formulated into an oral agent consisting of a solid composition, the agent can be formulated into a tablet, a pill, a capsule, powder, grain, granule or the like, and, for example, starch, lactose, white sugar, mannite, carboxymethylcellulose, corn starch, an inorganic salt or the like is utilized as a carrier. In addition, upon preparation of an oral agent, a binder, a disintegrant, a surfactant, a lubricant, a flowability-promoting agent, a corrective, a coloring agent, a flavor or the like may be further blended therewith. For example, when formulated into a tablet or a pill, preparations may be covered with a sugar-coating such as sucrose, gelatin or hydroxypropyl cellulose, or with a film of a substance which is soluble in stomach or intestine, as desired. When formulated into an oral agent consisting of a liquid composition, the preparation can be formulated into a pharmaceutically acceptable emulsion, solution, suspension, syrup or the like, and for example, purified water or ethanol is utilized as a carrier. Further, an adjuvant such as a wetting agent or a suspending agent, a sweetener, a flavor and an antiseptic may be added, as desired. Since the active ingredient of the present invention can exhibit a sufficient effect even by oral administration, from a viewpoint of simplicity of administration, it is preferable to formulate the preparation into a medicine for oral administration.

On the other hand, when formulated into a parenteral agent, the agent can be prepared by dissolving or suspending the aforementioned active ingredient of the present invention in distilled water for injection, a physiological saline, an aqueous glucose solution, a vegetable oil for injection, a sesame oil, a peanut oil, a soybean oil, a corn oil, propylene glycol, polyethylene glycol or the like as a diluent, and, if necessary, adding a disinfectant, a stabilizer, an isotonicifying agent, an analgesic agent or the like, according to a conventional method. Alternatively, a solid composition can also be prepared, to be dissolved in sterile water or a sterile solvent for injection prior to use.

An external agent includes solid, semi-solid or liquid preparations for transdermal administration or transmucosal (buccal, nasal) administration. In addition, a suppository is also included. For example, the external agent can be formulated as an emulsion such as an emulsion or a lotion, a liquid preparation such as an external tincture or a solution for transmucosal administration, an ointment such as an oily ointment or a hydrophilic ointment, and a patch for transdermal administration or transmucosal administration such as a film, a tape or a poultice, or the like.

The aforementioned various preparations can be appropriately produced respectively by a conventional method, utilizing a known pharmaceutical carriers or the like. In addition, the content of an active ingredient in such a preparation is not particularly limited, as far as it is such an amount that, in view of an administration form, an administration method or the like, the active ingredient can be administered in a dose range described later. The content of the active ingredient in the medicine of the present invention is around 0.1 to 100% by weight.

The antitumor agent, the apoptosis-inducing agent or the angiogenesis-suppressing agent of the present invention is administered by a suitable administration route according to a preparation form. An administration method is also not particularly limited, and may be via internal application, external application and injection. An injectant can be administered, for example, intravenously, intramuscularly, subcutaneously, intracutaneously or the like, and, in the case of an external agent, for example, a suppository may be administered by an appropriate administration method therefor.

A dose of the medicine of the present invention is appropriately set depending on a preparation form, an administration method, a purpose of use, and the age, weight and symptoms of a patient to be subjected to the administration of the medicine, and is not constant. Generally, when the compound represented by the general formula (1) is used as an active ingredient, the dose of the aforementioned active ingredient contained in a preparation is, for example, 0.1 μg to 500 mg/kg body weight, preferably 0.5 μg to 400 mg/kg body weight, and further preferably 1 μg to 300 mg/kg body weight per adult a day. In addition, when the aforementioned *Lyophyllum ulmarium*-derived composition (obtained by drying to solidness under reduced pressure) is used as an active ingredient, the dose is, for example, 0.1 μg to 1 g/kg body weight, preferably 0.5 μg to 750 mg/kg body weight, and further preferably 1 μg to 500 mg/kg body weight per adult a day. Since the dose of course varies depending on various conditions, an amount smaller than the aforementioned dose is sufficient in some cases, or an amount exceeding the above range is necessary in some cases. Administration may be performed in a single dose or divided into several doses in a day within the desired dose range. An administration period is also arbitrary. In addition, the medicine of the present invention may be orally administered as it is, or additionally, the medicine may be ingested routinely by adding the medicine to any beverage or food.

The food of the present invention is a food having an antitumor activity, an apoptosis-inducing activity or an angiogenesis-suppressing activity, containing a compound represented by the general formula (1), a derivative thereof, a pharmacologically acceptable salt thereof, and/or the aforementioned *Lyophyllum ulmarium*-derived composition, and is particularly useful in that homeostasis of a living body is maintained by ingesting or eating the food of the present invention. In addition, the food can be, for example, a health food (designated health food) with a label saying that the food is used for exhibiting desired effects due to the antitumor activity, the apoptosis-inducing activity or the angiogenesis-suppressing activity.

A process for producing the food of the present invention is not particularly limited. For example, blending, cooking, processing or the like may be carried out according to those of general foods, and the food may be produced by those processes. The resulting food has only to contain the aforementioned active ingredient of the present invention.

In the food of the present invention, "contain(ing)" means inclusion, addition and/or dilution. Here, "inclusion" refers to an embodiment in which the active ingredient used in the present invention is contained in a food, "addition" refers to an embodiment in which the active ingredient used in the present invention is added to a raw material of the food, and "dilution" refers to an embodiment in which a raw material of the food is added to the active ingredient used in the present invention.

The food of the present invention is not particularly limited. The food includes, for instance, processed agricultural and forest products, processed stock-raising products, processed marine products and the like, including processed grain products such as processed wheat products, processed starch products, processed premix products, noodles, macaronis, bread, bean jam, buckwheat noodles, wheat-gluten bread, rice noodle, *fen-tiao,* and packed rice cake; processed fat-and-oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, steamed fish paste, tubular roll of steamed fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); dairy products such as raw-material milk, cream, yogurt, butter, cheese, condensed milk, powder milk, and ice cream; processed vegetable- and fruit-products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor *(shochu),* vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, bottled or pouched foods such as rice topped with cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods (such as soups); spices; and the like.

In the food of the present invention, one or a plurality of the aforementioned active ingredient(s) are contained in, added to and/or diluted in the food. The shape of the food is not particularly limited, as far as the content of the active ingredient corresponds to a necessary amount for exhibiting the antitumor activity, the apoptosis-inducing activity or the angiogenesis-suppressing activity, and an orally ingestible shape such as tablet-like, granular or capsule-like shapes

may be included. Alternatively, glycerol or the like may be added to the aforementioned active ingredient, to obtain a health food in which the active ingredient is concentrated.

The content of the aforementioned active ingredient in the food of the present invention is not particularly limited, and can be appropriately selected from a viewpoint of exhibiting its function and activity. For example, when the compound represented by the general formula (1) is used, the content in the food is preferably 0.00001% by weight or more, more preferably 0.0001 to 10% by weight, and further preferably 0.0006 to 6% by weight. In addition, when the aforementioned *Lyophyllum ulmarium*-derived composition (obtained by drying to solidness under reduced pressure) is used, the content in the food is preferably 1% by weight or more, more preferably 5 to 95% by weight, and further preferably 10 to 90% by weight. In addition, the food of the present invention may be preferably ingested so that the aforementioned ingredient contained in the food is, for example, when the compound represented by the general formula (1) is used, 0.1 μg to 500 mg/kg body weight, preferably 0.5 μg to 400 mg/kg body weight, and further preferably 1 μg to 300 mg/kg body weight per adult a day. In addition, when the aforementioned *Lyophyllum ulmarium*-derived composition (obtained by drying to solidness under reduced pressure) is used, for example, the ingredient is ingested in an amount of 0.1 μg to 1 g/kg body weight, preferably 0.5 μg to 750 mg/kg body weight, and further preferably 1 μg to 500 mg/kg body weight per adult a day.

A tenth invention of the present invention provides a method of treating a disease in a subject in need of an antitumor action, an apoptosis-inducing action or an angiogenesis-suppressing action, comprising administering to the subject an effective amount of at least one substance selected from the group consisting of the compound represented by the aforementioned general formula (1), a derivative thereof and a pharmacologically acceptable salt thereof.

In the present specification, the subject is preferably, but not limited to, a human, and may be, for example, a farm-raised animal, or a pet animal. Examples of the farm-raised animal include livestock such as an equine, a bovid, a swine, a sheep, a goat, a camel and a llama, an experimental animal such as a mouse, a rat, a guinea pig and a rabbit, and poultry such as a chicken, a duck, a turkey and an ostrich. Examples of the pet animal include a dog and a cat.

In addition, as used herein, an effective amount is an amount of at least one substance (active ingredient) selected from the group consisting of the compound represented by the general formula (1), a derivative thereof, and a pharmacologically acceptable salt thereof, which generates an antitumor action, an apoptosis-inducing action or an angiogenesis-suppressing action when the active ingredient is administered to the subject, as compared with a subject to which the active ingredient has not been administered. A specific effective amount is appropriately set depending on an administration form, an administration method, a purpose of use and the age, weight, symptoms and the like of a subject, and is not constant. The amount is preferably 0.01 μg to 500 mg/kg body weight per human (e.g. adult) a day as expressed by an amount of the aforementioned active ingredient, as in the aforementioned medicine.

In the treating method of the present invention, an effective amount of at least one substance selected from the group consisting of the compound represented by the general formula (1), a derivative thereof and a pharmacologically acceptable salt thereof may be administered as it is to the aforementioned subject, or may be administered as the aforementioned medicine or food. In addition, an administration method is not limited, and, for example, it may be administered orally or by injection or the like, as in the aforementioned medicine.

According to the treating method of the present invention, a disease to be treated with the aforementioned medicine or food of the present invention can be treated, and, for example, the effect of treating cancer or suppressing progression or metastasis of cancer can be exhibited.

When SBS used as an active ingredient in the present invention is orally administered in an amount of 25 mg/mouse/day, toxicity is not observed.

## EXAMPLES

The present invention will be explained in detail hereinbelow by way of Examples, but the present invention is not limited to these Examples by any means. In the description of Examples, the term "tumor-suppressing activity" is used as a term synonymous with the antitumor activity.

### Preparation Example 1

Five liters of ethyl acetate was added to 250 g of dry powder of a fruit body *of Lyophyllum ulmarium* M-8171 (FERM BP-1415), and the mixture was stirred at room temperature for 3 hours. Thereafter, filtration (No. 2 filter manufactured by Advantec) was carried out, and the residue was removed, to obtain 5 L of a filtrate. One-hundred fifty grams of silica gel 60 (0.063 to 0.200 mm) (manufactured by Merck) was washed with about 800 ml of ethyl acetate, and filled into a glass column having a diameter of 5 cm to obtain a silica gel column. Thereafter, the filtrate was calmly poured into the silica gel column, and the column was washed with 3 L of ethyl acetate, to remove unadsorbed substances. Elution was then carried out with 3 L of acetone, to give 3 L of an eluate. The eluate was dried to solidness under reduced pressure, and 100 ml of 100% ethanol was added thereto. The dried product was dried again to solidness under reduced pressure, to obtain 3.6 g of an acetone-eluted fraction with acetone removed therefrom.

As a result of analysis of NMR and LC-MS, it was confirmed that the fraction contained the compounds represented by the formulas (2) to (11), and the major constitutional component thereof is the compound represented by the formula (3) (SBS).

Preparation Example 2

In the same manner as in Preparation Example 1 except that *Lyophyllum ulmarium was Lyophyllum ulmarium* K-0259 (FERM P-12981), 1.1 g of an acetone-eluted fraction was obtained. As a result of analysis of NMR and LC-MS, it was confirmed that the fraction contained the compounds represented by the formulas (2) to (11).

Example 1

As ICR mice (Japan SLC, Inc.), 5-week old female mice were purchased, and the animals were used at 6 weeks old. Sarcoma-180 (hereinafter, S-180) tumor cells were transplanted into an abdominal cavity of the ICR mice to give an ascites, and the ascites was passaged by transplanting into other mice every 7 days. An ascites on day 7 after passage was collected, washed with a phosphate buffered saline by centrifugation, and suspended in the same buffer. The number of cells was counted, and the suspension was adjusted to have a density of $5 \times 10^7$ cells/mL. Into ICR mice at right abdominal part was subcutaneously transplanted 0.1 mL of the cell suspension, and the size of a solid tumor after 7 days was measured. Mice were grouped so that the average size of the tumor became equal in each group, and one group included 10 animals. An acetone-eluted fraction derived from *Lyophyllum ulmarium* prepared in Preparation Example 1 was dissolved in 100% ethanol, and the solution was blended with a standard powder feed CE-2, which was given to the mice. The feed was used so that the dose in terms of a *Lyophyllum ulmarium* powder was equal to that in the case where the powder was given by blending with a powder feed CE-2 at a ratio of 10% by volume. An actual dose (acetone-eluted fraction) was to be about 250 mg/kg/day. Only CE-2 was given to a control group. The size of the tumor was measured after four weeks from transplantation of S-180. In addition, as the size of the tumor, a length and a width were measured, to obtain the volume according to the following equation, followed by comparison.

$$\text{Tumor volume (mm}^3\text{)} = \text{(length)} \times \text{(width)}^2/2$$

The tumor-suppressing activity was calculated according to the following equation.

$$\text{Tumor-suppressing activity (\%)} = \text{(tumor volume of a control group - tumor volume of a group administered with acetone-eluted fraction)/tumor volume of a control group} \times 100$$

[Table 1]

Table 1

| Experiment Number | Mice | Tumor Volume (Mean ± SD) | Tumor-Suppressin g Activity (%) |
|---|---|---|---|
| 1 | CE-2 (Control) | 1862 ± 542 | |
| | Acetone-Eluted Fraction (about 250 mg/kg) | 1171 ± 270 | 37.1 |
| 2 | CE-2 (Control) | 1437 ± 321 | |
| | Acetone-Eluted Fraction (about 250 mg/kg) | 619 ± 132 | 56.9 |

Results of duplicate experiments are shown in Table 1. In both experiments, as compared with the control group, suppression of growth of an S-180 solid tumor was observed in a group to which the *Lyophyllum ulmarium*-

*derived* acetone-eluted fraction had been administered.

Example 2

As $CDF_1$ mice (Japan SLC, Inc.), 6-week old female mice were purchased, and the animals were used at 7 weeks old. IMC carcinoma (hereinafter, IMC) tumor cells were transplanted into an abdominal cavity of the $CDF_1$ mice to give an ascites, and the ascites was passaged by transplantion into other mice every 7 days. An ascites on day 7 after passage was collected and washed with a phosphate buffered saline by centrifugation. Thereafter, the washed cells were suspended in the same buffer, and the number of cells was counted. The suspension was adjusted to have a density of $5 \times 10^7$ cells/mL. Into $CDF_1$ mice at a right abdominal part was subcutaneously transplanted 0.1 mL of the resulting cell suspension, and the size of a solid tumor on day 7 after transplantation was measured. Mice were grouped so that the average size of the tumor became equal in each group, and one group included 10 animals. The acetone-eluted fraction derived from *Lyophyllum ulmarium* prepared in Preparation Example 1 was dissolved in 100% ethanol, and the solution was blended with a standard powder feed CE-2, which was given to the mice. The feed was used so that a dose in terms of a *Lyophyllum ulmarium* powder was equal to that in the case where the powder was given by blending with a powder feed CE-2 at a ratio of 10% by volume. An actual dose was to be about 250 mg/kg/day. Only CE-2 was given to a control group. The size of the tumor was measured after four weeks from transplantation of IMC. In addition, as the size of a tumor, a length and a width were measured, and the volume and the tumor-suppressing activity were calculated and compared as in Example 1. Results are shown in Table 2.

[Table 2]

Table 2

| Mice | Tumor Volume (Mean ± SD) | Tumor-Suppressing Activity (%) |
|---|---|---|
| CE-2 (Control) | 3081 ± 724 | |
| Acetone-Eluted Fraction (about 250 mg/kg) | 1505 ± 246 | 51.2 |

As compared with the control group, suppression of growth of an IMC solid tumor was observed in a group to which the *Lyophyllum ulmarium*-derived acetone-eluted fraction had been administered. IMC carcinoma is inbred to $CDF_1$ mice, and it was revealed that the *Lyophyllum ulmarium*-derived acetone-eluted fraction also suppresses growth of such an inbred tumor.

Example 3

As HL-60 cells, cells obtained by passaging twice a week using an RPMI-1640 medium with 10% fetal bovine serum and Gentamycin added at a final concentration of 50 μg/ml were used. The cells were washed twice with a serum-free RPMI-1640 medium and suspended in the same medium, and the number of cells was counted. The cells were diluted so as to have a density of $2 \times 10^6$ cells/mL, and each 1 mL of the suspension was dispensed into a 24-well culture plate. The acetone-eluted fraction prepared in Preparation Example 1 was added thereto so as to have a concentration of 0.2 μM, 2 μM or 20 μM, and the cells were cultured in an incubator at 37°C for 6 hours. For a negative control, a vehicle (1% aqueous ethanol solution) was used, and for a positive control, 1 μg/mL of Actinomycin D was used. After culturing for 6 hours, cells were recovered by centrifugation. Detection of apoptosis cells was performed as follows: Cells were fixed in 4% paraformaldehyde (pH 7.4) at room temperature for 15 minutes. Thereafter, the cells were suspended in 70% ethanol, and the suspension was allowed to stand at -20°C overnight. The cells were washed with a phosphate buffered saline (PBS (-)). Thereafter, a ribonuclease A solution was added thereto, and the cells were incubated at 37°C for about 20 minutes. After recovery of cells by centrifugation, a propidium iodide solution was added thereto to stain cells, and the ratio of apoptosis cells was measured with a flow cytometer (manufactured by Beckmann Coulter, Inc.). An aliquot was taken on a glass slide, and it was confirmed under a fluorescent microscope that apoptosis was caused. Results are shown in Fig.1.

Example 4

As C57BL/6 mice (Japan SLC, Inc.), 6-week old female mice were purchased, and the animals were used at 7 weeks old. Assessment of angiogenesis using Matrigel was performed according to the method of Passaniti et al.(Lab. Invest. 67:519-528 (1992)). That is, the acetone-eluted fraction prepared in Preparation Example 1 was added, at a concentration shown in Table 3, to Matrigel (manufactured by Becton, Dickinson and Company) to which acidic fibroblast growth factor (aFGF) and heparin had been added at a concentration of 1 μg/mL and 64 U/mL, respectively, and 0.5

mL of the solution was subcutaneously transplanted into the mice at a right abdominal part. As a control, a vehicle (1% aqueous ethanol solution) used for dissolving an acetone-eluted fraction was added in place of an acetone-eluted fraction. After five days, Matrigel was isolated. The degree of angiogenesis was compared by homogenizing the isolated Matrigel and measuring the amount of hemoglobin. For measuring the amount of hemoglobin, Hemoglobin B-Test Wako (manufactured by Wako Pure Chemical Industries, Ltd.) was used. Results are shown in Table 3.

[Table 3]

Table 3

| Sample | Mice (Number) | Hemoglobin Concentration (g/mL) |
|---|---|---|
| Control | 5 | 1641 |
| Acetone-Eluted Fraction 20 μM | 4 | 642 |

As described above, by acting the acetone-eluted fraction, the hemoglobin concentration was suppressed. In other words, this is because the amount of blood entering Matrigel was suppressed due to suppression of angiogenesis into Matrigel.

Example 5

As C57BL/6 mice (Japan SLC, Inc.), 6-week old female mice were purchased, and the animals were used at 7 weeks old. Assessment of angiogenesis using Matrigel was performed in the same manner as in Example 4. That is, Matrigel with aFGF and heparin added thereto was subcutaneously injected into the mice, and Matrigel was isolated after five days, followed by measurement of the amount of hemoglobin. The *Lyophyllum ulmarium*-derived acetone-eluted fraction prepared in Preparation Example 1 was lyophilized, and blended with a standard powder feed CE-2, which was given to the mice. The feed was used so that a dose in terms of a *Lyophyllum ulmarium* powder was equal to that in the case where the powder was given by blending with a powder feed CE-2 at a ratio of 10% by volume. An actual dose was to be about 220 mg/kg/day. Only CE-2 was given to a control group. Results are shown in Table 4.

[Table 4]

Table 4

| Sample | Mice (Number) | Hemoglobin Concentration (g/mL) |
|---|---|---|
| Control | 5 | 1641 |
| Administrated with Feed Blended with Acetone-Eluted Fraction | 3 | 527 |

When the acetone-eluted fraction was blended with a feed and administered, angiogenesis to Matrigel was also suppressed. It demonstrated the angiogenesis-suppressing action by the acetone-eluted fraction. In addition, it was also suggested that the suppressing action on tumor growth observed upon administration of a feed blended with the acetone-eluted fraction may be an action generated by suppressing angiogenesis of a tumor.

Example 6

U937 cells (human lymphoma cells) were suspended in an RPMI-1640 medium with 10% fetal bovine serum added thereto, and the number of cells was counted. The suspension was adjusted to have a density of $5 \times 10^4$ cells/mL. Each 2 mL of the suspension was dispended into a plate for cell culture. The acetone-eluted fraction prepared in Preparation Example 1 was diluted with ethanol to 1 or 2 mM, and each 20 μL was added thereto to have a final concentration of 10 or 20 μM, respectively. Only ethanol was added to a control. Incubation was performed under the conditions of 37°C and 5% $CO_2$. After 6 hours, after 24 hours, after 48 hours or after 72 hours, cells were recovered, and stained with 0.3% by weight trypan blue, and the number of whole cells and the number of dead cells were counted. Results of the counting of only the number of live cells are shown in Table 5. In addition, results of a survival ratio of the cells calculated by the following equation are shown in Table 6. An experiment was performed at N=4, and results are shown as an average. In addition, a survival ratio was calculated by the following equation:

$$\text{Survival ratio (\%)} = (\text{number of live cells / number of whole cells}) \times 100$$

[Table 5]

Table 5 Number of Live U937 Cells (10.0 × $10^4$ cells at the initiation)

| Amount of Added Sample (μM) | Number of Live Cells (× $10^4$ cells) | | | |
|---|---|---|---|---|
| | Culture Period (hours) | | | |
| | 6 | 24 | 48 | 72 |
| Control | 12.2 | 18.3 | 37.5 | 86.3 |
| 10 | 9.6 | 6.2 | 4.6 | 3.8 |
| 20 | 10.2 | 0.5 | 0 | 0 |

[Table 6]

Table 6 Cytotoxic Activity against U937

| Amount of Added Sample (μM) | Survival Ratio (%) | | | |
|---|---|---|---|---|
| | Culture Period (hours) | | | |
| | 6 | 24 | 48 | 72 |
| Control | 97.5 | 96.7 | 97.0 | 97.8 |
| 10 | 97.4 | 55.5 | 33.6 | 30.9 |
| 20 | 96.0 | 6.0 | 0 | 0 |

As described above, it was revealed that the acetone-eluted fraction exhibits a cytotoxic activity, which is a kind of an antitumor activity, on human lymphoma cells.

Example 7

MKN45 cells (human stomach cancer cells) were suspended in an RPMI-1640 medium with 10% fetal bovine serum added thereto, and the number of cells was counted. The suspension was adjusted to have a density of 5 × $10^4$ cells/mL. Each 2 mL of the suspension was dispensed into a plate for cell culture and cultured overnight under the conditions of 37°C and 5% $CO_2$. Thereafter, the medium was removed, and 1 mL of a fresh medium was added thereto. The acetone-eluted fraction prepared in Preparation Example 1 was diluted with ethanol to 2 or 4 mM, and each 10 μL of the suspension was added thereto to have a final concentration of 20 or 40 μM, respectively. As a control, only ethanol was added. Incubation was performed under the conditions of 37°C and 5% $CO_2$. After 6 hours, after 24 hours, after 48 hours or after 72 hours, the cells were recovered by trypsin-EDTA treatment and stained with 0.3% by weight of trypan blue. The number of whole cells and the number of dead cells were counted, and a survival ratio was calculated in the same manner as in Example 6. Results of the counting of only the number of live cells are shown in Table 7. In addition, results of a survival ratio are shown in Table 8. An experiment was performed at N=4, and results are shown as an average.

[Table 7]

Table 7 Number of Live MKN45 Cells (10.0 × $10^4$ cells at the initiation)

| Amount of Added Sample (μM) | Number of Live Cells (x $10^4$ cells) | | | |
|---|---|---|---|---|
| | Culture Period (hours) | | | |
| | 6 | 24 | 48 | 72 |
| Control | 11.8 | 20.7 | 43.6 | 71.5 |
| 20 | 7.5 | 8.2 | 6.3 | 4.6 |
| 40 | 6.2 | 1.8 | 0.2 | 0.1 |

[Table 8]

Table 8 Cytotoxic activity against MKN45 Cells (survival ratio at the initiation: 100%)

| Amount of Added Sample (μM) | Survival Ratio (%) | | | |
|---|---|---|---|---|
| | Culture Period (hours) | | | |
| | 6 | 24 | 48 | 72 |
| Control | 96.6 | 98.0 | 97.6 | 97.4 |
| 20 | 95.4 | 90.0 | 81.6 | 63.5 |
| 40 | 82.2 | 35.4 | 2.3 | 1.0 |

As described above, it was revealed that the acetone-eluted fraction exhibits a cytotoxic activity, which is a kind of an antitumor activity, also on human stomach cancer cells.

Example 8

S-180 cells (mouse cancer cells) or IMC cells (mouse cancer cells) which were ascites-passaged using ICR mice or $CDF_1$ mice were, respectively, passaged by transplantation. After seven days, an ascites was collected and used. The cells were washed with a phosphate buffered saline by centrifugation and suspended into an RPMI-1640 with 10% fetal bovine serum added thereto. The number of cells was counted, and the suspension was adjusted to have a density of $1 \times 10^6$ cells/mL. Each 1 mL of the suspension was dispensed into Petri dishes. The acetone-eluted fraction prepared in Preparation Example 1 was diluted with ethanol to 1, 2 or 4 mM, and each 10 μL of the suspension was added thereto to have a final concentration of 10, 20 or 40 μM, respectively. Only ethanol was added to a control. Incubation was performed under the conditions of 37°C and 5%$CO_2$. After 6 hours, after 24 hours, after 48 hours or after 72 hours, the cells were recovered and stained with 0.2% trypan blue. The number of whole cells and the number of dead cells were counted, and a survival ratio was calculated in the same manner as in Example 6 (Table 9 and Table 10).
[Table 9]

Table 9 Cytotoxic Activity against S-180 (survival ratio at the initiation: 98.5%)

| Amount of Added Sample (μM) | Survival Ratio (%) | | | |
|---|---|---|---|---|
| | Culture Period (hours) | | | |
| | 6 | 24 | 48 | 72 |
| Control | 99.2 | 97.5 | 94.6 | 92.8 |
| 10 | 99.7 | 94.4 | 8.1 | 18.1 |
| 20 | 99.7 | 25.4 | 0.0 | 0.0 |
| 40 | 7.0 | 0.0 | 0.0 | 0.0 |

[Table 10]

Table 10 Cytotoxic Activity against IMC (survival ratio at the initiation: 98.2%)

| Amount of Added Sample (μM) | Survival Ratio (%) | | | |
|---|---|---|---|---|
| | Culture Period (hours) | | | |
| | 6 | 24 | 48 | 72 |
| Control | 99.0 | 98.7 | 95.3 | 78.5 |
| 10 | 100 | 96.7 | 71.7 | 9.3 |
| 20 | 92.0 | 18.3 | 2.1 | 0.9 |
| 40 | 44.6 | 2.3 | 1.1 | 1.8 |

As described above, it was revealed that the acetone-eluted fraction exhibits a cytotoxic activity, which is a kind of an antitumor activity, also on mouse cancer cells.

Preparation Example 3

Ethyl acetate was added to 300 g of powder of a fruit body of *Lyophyllum ulmarium* M-8171 to a total amount

of 5 L, and the mixture was stirred at room temperature for 3 hours, to extract ethyl acetate-soluble components. Insolubles were removed by suction-filtration, to obtain an ethyl acetate-soluble fraction. The fraction was applied to a Silicagel 60 column (300-mL volume), and 3 L of ethyl acetate was poured to elute an unadsorbed fraction. Thereafter, 3 L of acetone was poured to recover an acetone-eluted fraction. After concentration under reduced pressure, ethanol was added thereto. The mixture was concentrated to dryness under reduced pressure again, and the weight was measured. This procedure was performed ten times, and finally, 35.2g of an acetone-eluted fraction was obtained from 3 kg of powder of a fruit body of *Lyophyllum ulmarium.* Five-hundred milligrams of the fraction was applied to a Silicagel 60 column, and a gradient of ethyl acetate: acetone (50:50) to (0:100) recovered a main component. After removal of a solvent by concentration under reduced pressure, the concentrate was dissolved in a mixed solvent of methanol: ethanol: water (3:4:4), and SBS was taken by high-performance liquid chromatography using an ODS column (CAPCELLPAK $C_{18}$ UG120Å 5 μm ϕ20 × 250 mm) under the conditions of a flow rate of 10 ml/min and a detection wavelength of 210 nm. As an eluting solvent, a solvent with SBS dissolved therein was used. The taken SBS was concentrated to dryness, to obtain 260 mg of purified SBS.

Example 9

HL-60 cells (human leukemia cells) were suspended in an RPMI-1640 with 10% fetal bovine serum added thereto, and the number of cells was counted. The suspension was adjusted to have a density of $5 \times 10^5$ cells/mL. Each 2 mL of the suspension was dispensed into Petri dishes. SBS prepared in Preparation Example 3 was diluted with dimethyl sulfoxide to 2 mM, and each 20 μL of the suspension was added thereto to have a final concentration of 20 μM. Only dimethyl sulfoxide was added to a control. Incubation was performed for 6 hours under the conditions of 37°C and 5% $CO_2$, and the cells were recovered, and washed with a phosphate buffered saline (PBS) by centrifugation. Four-percent paraformaldehyde was added to the cells, and the cells were allowed to stand at room temperature for 30 minutes to be fixed. After centrifugation, the supernatant was removed, and a PBS was added thereto, and the mixture was centrifuged and washed, and the cells were suspended in 200 μL of a PBS. Five-hundred microliters of 0.05 mg/mL propidium iodide was added thereto, and the mixture was incubated at 4°C for 10 minutes. Thereafter, the number of whole cells and the number of cells which had undergone apoptosis were measured, and a ratio of induction of apoptosis was calculated according to the following equation (Table 11).

$$\text{Apoptosis-induction ratio (\%)} = (\text{number of cells wherein apoptosis is induced / number of whole cells}) \times 100$$

[Table 11]

Table 11

| Sample | Apoptosis-Inducing Ratio (%) |
|---|---|
| Control | 9.3 |
| SBS (20 μM) | 30.1 |

As described above, it was revealed that the purified SBS also induces apoptosis in cancer cells.

Example 10

As $CDF_1$ mice (Japan SLC, Inc.), 6-week old female mice were purchased, and the animals were used at 7 weeks olds. As IMC carcinoma (hereinafter, IMC) tumor cells were transplanted into an abdominal cavity of the $CDF_1$ mice to make an ascites, and transplanted into other mice and passaged every 7 days. An ascites on day 7 from transplantation passage was taken, and suspended in a phosphate buffered saline. The suspension was centrifuged, and the precipitates were suspended in the same buffer. The number of cells was counted. The suspension was adjusted to have a density of $5 \times 10^7$ cells/mL, and 0.1 mL of the suspension was subcutaneously transplanted into $CDF_1$ mice at a right abdominal part. Seven days after transplantation, take of a tumor was confirmed, and the size of a solid tumor was measured. Mice were grouped so that the average size of the tumor became equal in each group, and one group included 12 or 11 animals. SBS prepared in Preparation Example 3 was dissolved in ethanol, and the suspension was

diluted with peanut oil so as to have an ethanol concentration of 10%, and was orally administered to the mice. An actual dose was to be about 15.6 mg/kg/day. Peanut oil containing 10% of ethanol was administered to a control group. Three weeks after transplantation of an IMC tumor, the length and the width of the tumor was measured, and the tumor volume and the tumor-suppressing activity were calculated and compared in the same manner as in Example 1. Results are shown in Table 12.

[Table 12]

Table 12

| Administered Sample | Tumor Volume (Mean ± SD) | Tumor-Suppressing Activity (%) |
|---|---|---|
| CE-2 (Control) | 992.9 ± 179.6 | - |
| SBS 15.6 (mg/kg) | 534.5 ± 92.3 | 46.2 |

As described above, in the animal group with SBS orally administered thereto, significant suppression of tumor growth was observed as compared with the control group.

INDUSTRIAL APPLICABILITY

According to the present invention, an antitumor agent, an apoptosis-inducing agent, or an angiogenesis-suppressing agent containing as an active ingredient polyterpene isolated from an inexpensive edible basidiomycete or a composition derived from *Lyophyllum ulmarium,* and a food having an antitumor activity, an apoptosis-inducing activity or an angiogenesis-suppressing activity can be obtained. Therefore, the present invention is particularly useful, especially in the field of a medicine and a health food.

**Claims**

1. An antitumor agent, an apoptosis-inducing agent or an angiogenesis-suppressing agent, **characterized in that** the agent contains as an active ingredient at least one substance selected from the group consisting of a compound represented by the following general formula (1), a derivative thereof, and a pharmacologically acceptable salt thereof,

[Chemical Formula 1]

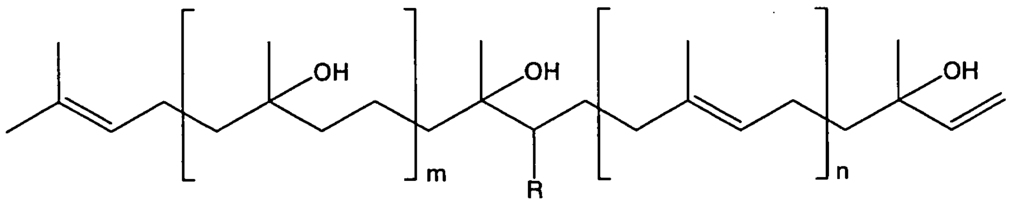

(1)

wherein m represents an integer of 1 to 9, n represents an integer of 1 to 7, provided that **m** + **n=5** to 11; and **R** represents a hydrogen atom or a hydroxyl group.

2. The antitumor agent, the apoptosis-inducing agent or the angiogenesis-suppressing agent according to claim 1, wherein the compound represented by the general formula (1) is at least one compound represented by the following formulas (2) to (11).

[Chemical Formula 2]

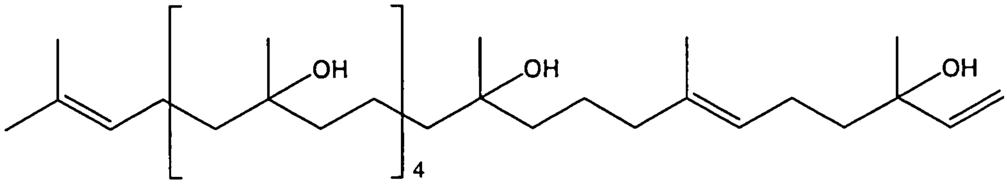

(2)

[Chemical Formula 3]

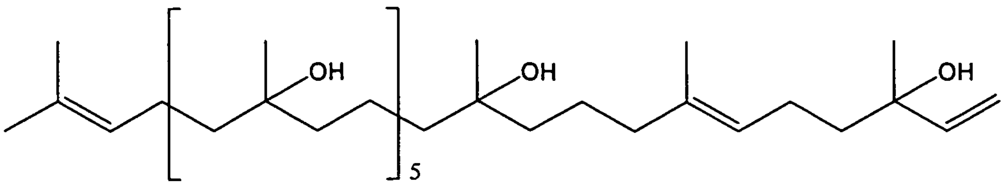

(3)

[Chemical Formula 4]

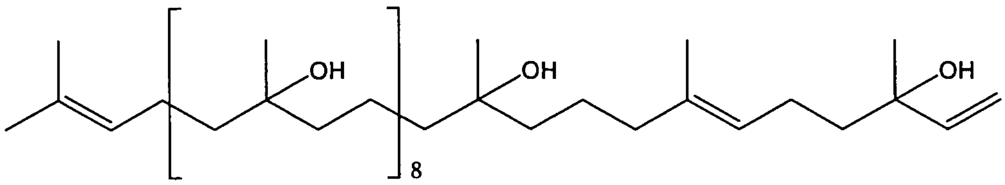

(4)

[Chemical Formula 5]

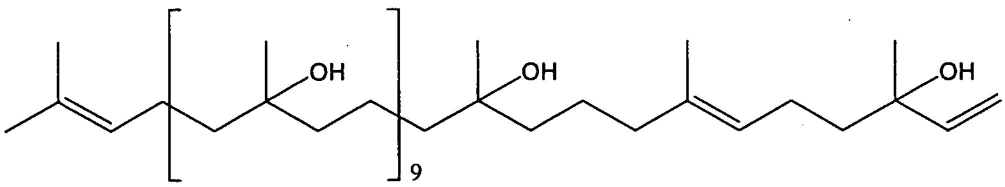

(5)

[Chemical Formula 6]

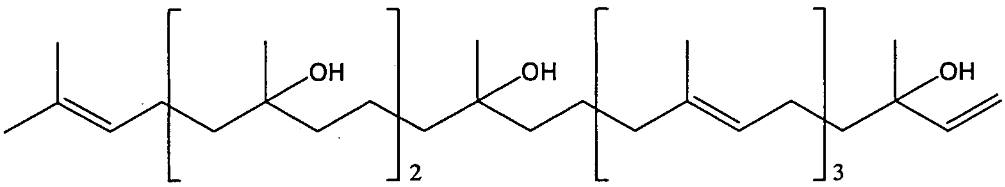

(6)

[Chemical Formula 7]

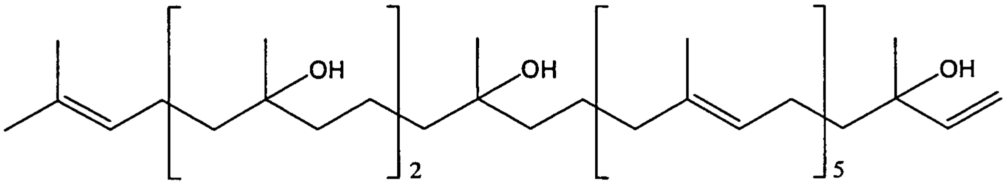

(7)

[Chemical Formula 8]

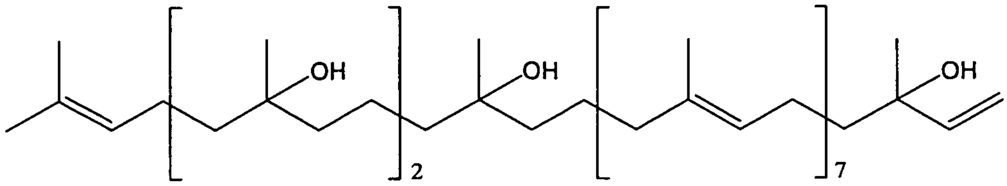

(8)

[Chemical Formula 9]

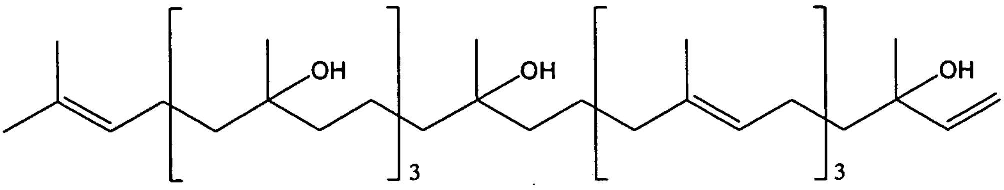

(9)

[Chemical Formula 10]

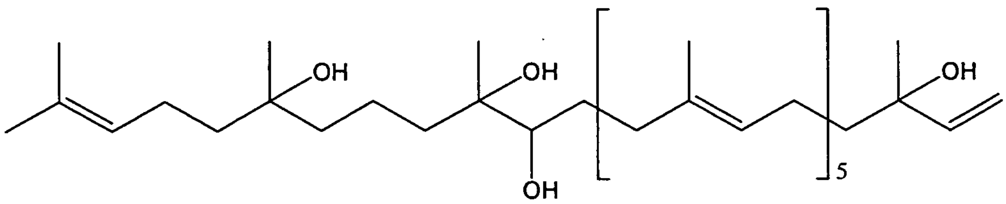

(10)

[Chemical Formula 11]

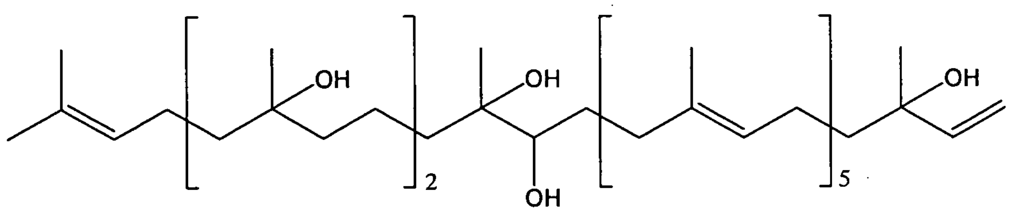

(11)

3. A food having an antitumor activity, an apoptosis-inducing activity or an angiogenesis-suppressing activity, **characterized in that** the food contains at least one substance selected from the group consisting of a compound represented by the general formula (1), a derivative thereof, and a pharmacologically acceptable salt thereof.

4. The food according to claim 3, wherein the compound represented by the general formula (1) is at least one compound represented by the formulas (2) to (11).

5. An antitumor agent, an apoptosis-inducing agent or an angiogenesis-suppressing agent, **characterized in that** the agent contains as an active ingredient a *Lyophyllum ulmarium*-derived composition containing at least one substance selected from the group consisting of compounds represented by the formulas (2) to (11), a derivative thereof, and a pharmacologically acceptable salt thereof.

6. The antitumor agent, the apoptosis-inducing agent or the angiogenesis-suppressing agent according to claim 5, **characterized in that** the *Lyophyllum ulmarium*-derived composition is a composition obtained by adsorbing an ethyl-acetate extract of *Lyophyllum ulmarium* onto a silica gel column, followed by elusion.

7. A food having an antitumor-activity, an apoptosis-inducing activity or an angiogenesis-suppressing activity, **characterized in that** the food contains a *Lyophyllum ulmarium*-derived composition containing at least one substance selected from the group consisting of compounds represented by the formulas (2) to (11), a derivative thereof, and a pharmacologically acceptable salt thereof.

8. The food according to claim 7, **characterized in that** the *Lyophyllum ulmarium-derived* composition is a composition obtained by adsorbing an ethyl-acetate extract of *Lyophyllum ulmarium* onto a silica gel column, followed by elusion.

9. *ALyophyllum ulmarium*-derived composition containing at least one substance selected from the group consisting of compounds represented by the formulas (2) to (11), a derivative thereof and a pharmacologically acceptable salt thereof, wherein the composition is obtained by adsorbing an ethyl-acetate extract of *Lyophyllum ulmarium* onto a silica gel column, followed by elusion.

10. A method of treating a disease in a subject in need of an antitumor action, an apoptosis-inducing action or an angiogenesis-suppressing action, comprising administering to the subject an effective amount of at least one substance selected from the group consisting of a compound represented by the aforementioned general formula (1), a derivative thereof, and a pharmacologically acceptable salt thereof.

11. Use of at least one substance selected from the group consisting of a compound represented by the aforementioned general formula (1), a derivative thereof, and a pharmacologically acceptable salt thereof, for manufacturing an antitumor agent, an apoptosis-inducing agent or an angiogenesis-suppressing agent.

[Fig. 1]

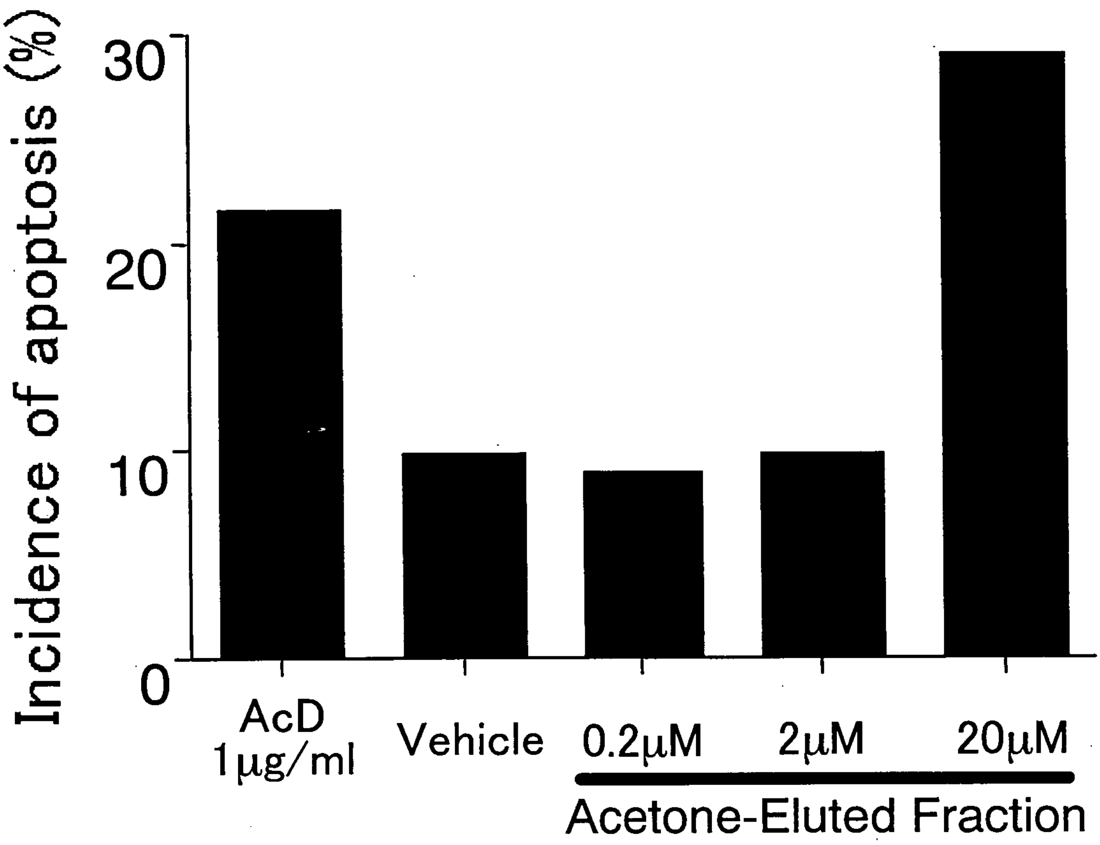
Incidence of apoptosis (%)
30
20
10
0
AcD
1μg/ml
Vehicle
0.2μM
2μM
20μM
Acetone-Eluted Fraction

**INTERNATIONAL SEARCH REPORT**

International application No.
PCT/JP2005/009895

A. CLASSIFICATION OF SUBJECT MATTER
Int.Cl$^7$ A61K31/047, 35/84, A61P35/00

According to International Patent Classification (IPC) or to both national classification and IPC

B. FIELDS SEARCHED
Minimum documentation searched (classification system followed by classification symbols)
Int.Cl$^7$ A61K31/047, 35/84, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY(STN), CAPLUS(STN), BIOSIS(STN), MEDLINE(STN), EMBASE(STN), JSTPLUS(JOIS), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| P,X | Chang, J.S. et al., Inhibition of cell cycle progression on HepG2 cells by hypsiziprenol $A_9$, isolated from Hypsizigus marmoreus, Cancer Letters, August 2004, Vol.212, pages 7 to 14, Results | 1-9,11 |
| X | JP 2860963 B2 (Takara Shuzo Kabushiki Kaisha), 11 December, 1998 (11.12.98), Column 3, lines 8 to 11; examples 1 to 3; experimental example 3 (Family: none) | 1-9,11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 June, 2005 (09.06.05) | 19 July, 2005 (19.07.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/009895

C (Continuation). DOCUMENTS CONSIDERED TO BE RELEVANT

| Category* | Citation of document, with indication, where appropriate, of the relevant passages | Relevant to claim No. |
|---|---|---|
| X | SAWABE, A., et al., Structural analyses of a precursory substance of bitterness: new polyisoprenepolyols isolated from an edible mushroom (Hypsizigus marmoreus) by fast atom bombardment mass spectrometry, J.Agric.Food. Chem., 1999, Vol.47, pages 588 to 593, Introduction, tables 1, 3 | 1-9,11 |
| A | JP 5-306233 A (Nagano-Ken Keizai Jigyo Nogyo Kyodo Kumiai Rengokai), 19 November, 1993 (19.11.93), (Family: none) | 1-9,11 |

Form PCT/ISA/210 (continuation of second sheet) (January 2004)

INTERNATIONAL SEARCH REPORT

International application No.
PCT/JP2005/009895

**Box No. II Observations where certain claims were found unsearchable (Continuation of item 2 of first sheet)**

This international search report has not been established in respect of certain claims under Article 17(2)(a) for the following reasons:

1. [X] Claims Nos.: 10
because they relate to subject matter not required to be searched by this Authority, namely:
The invention according to claim 10 relates to a method of treating a disease with a need for an antitumor effect, an apoptosis-inducing effect or an angiogenesis inhibitory effect with the use of triterpene and pertains to methods for treatment of the human body by (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

INTERNATIONAL SEARCH REPORT

International application No.
PCT/JP2005/009895

Continuation of Box No.II-1 of continuation of first sheet(2)

therapy. Thus, it relates to a subject matter which this International Searching Authority is not required, under the provisions of Article 17(2)(a)(i) of the PCT and Rule 39.1(iv) of the Regulations under the PCT, to search.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5306233 A
- WO 0151070 A
- JP 4104795 A


### Non-patent literature cited in the description


- **SAWABE A.** *Journal of Mass Spectrometry,* 1996, vol. 31, 921-925
- **SCHWARZ R.E.** *J. Surg. Res.,* 2004, vol. 120 ((1)), 64-72
- **TAKAKU T.** *J. Nutr.,* 2001, vol. 131 ((5)), 1409-1413
- **PASSANITI et al.** *Lab. Invest.,* 1992, vol. 67, 519-528